# FASCICULE DE BREVET EUROPEEN

(11) **EP 0 679 101 B1**
(45) Date de publication et mention de la délivrance du brevet: **12.03.1997**
(21) Numéro de dépôt: 92915621.4
(22) Date de dépôt: 02.07.1992
(51) Int. Cl.: A61M 1/36, A61K 47/46

(54) **APPAREIL POUR FAIRE PENETRER DES MEDICAMENTS DANS DES GLOBULES ROUGES**
APPARAT ZUR EINBRINGUNG VON MEDIKAMENTEN IN ROTE BLUTKÖRPERCHEN
APPARATUS FOR DELIVERING DRUGS INTO RED BLOOD CORPUSCLES

(30) Priorité: 03.07.1991 FR 9108302
(43) Date de publication de la demande: 02.11.1995
(73) Titulaire: COMMUNAUTE EUROPEENNE, L-2920 Luxembourg (LU)
(72) Inventeur: PAGES, Etienne, F-37550 Saint-Avertin (FR); ROPARS, Claude, F-37550 Saint-Avertin (FR); BAILLEUL, Christophe, F-93370 Montfermeil (FR)
(74) Mandataire: Warcoin, Jacques
(86) Numéro de dépôt international: FR9200623
(87) Numéro de publication internationale: WO9300940

(56) Documents cités:
- EP-A- 0 292 076
- FR-A- 2 529 463
- US-A- 3 399 536
- US-A- 4 327 710
- US-A- 4 668 214

## Description

La présente invention concerne un dispositif permettant la mise en oeuvre de la technique dite de "lyse-rescellement" qui permet d'incorporer des principes actifs dans les globules rouges.

La technique de lyse-rescellement est décrite dans les brevets EP 101341 US 4752586 et US 4652449, et son principe ne sera pas redécrit en détail.

Dans la technique dite de "lyse-rescellement" on alimente en continu le compartiment primaire d'un premier élément de dialyse avec une suspension aqueuse d'érythrocytes, le compartiment secondaire contenant une solution aqueuse hypotonique par rapport à la suspension d'érythrocytes afin de lyser les érythrocytes. Le lysat d'éythrocytes est alors en contact avec la substance présentant une activité biologique et afin de resceller la membrane des érythrocytes, on augmente la pression osmotique et/ou oncotique du lysat érythrocytaire après sa mise en contact avec la substance à activité biologique.

Dans ce mode de mise en oeuvre préféré de ce procédé, le rescellement est effectué dans un récipient séparé bien qu'il soit possible, comme cela est décrit dans le brevet, de pratiquer le rescellement en utilisant successivement deux bobines de dialyse.

De façon plus précise dans ce type de procédé, le culot globulaire est obtenu par centrifugation et décantation du plasma, qui est conservé à 4°C en vue du traitement. Les globules rouges subissent une première série de lavages. Le premier lavage en sérum physiologique permet d'obtenir un concentré globulaire dont le "buffy-coat" (globules blancs, plaquettes) est éliminé. Le concentré de globules rouges est alors mis en contact avec l'IHP (inositol hexaphosphate) par exemple au cours des deux lavages suivants.

En milieu hypotonique, le globule rouge gonfle jusqu'à un volume qui peut être égal à 175 % de sa valeur initiale (ROPARS et Coll., 1986). C'est à ce stade qu'apparaissent des pores de quelques centaines d'angströms. Il y a alors échange entre les milieux extra-cellulaire et intra-cellulaire notamment pour la substance a internaliser. Après addition d'une solution hypertonique dans l'hémolysat, l'isotonicité est restaurée, les pores se referment et emprisonnent l'IHP par exemple dans le globule rouge. Suit une phase d'incubation en solution régénératrice, nécessaire aux globules rouges chargés pour retrouver des caractéristiques de perméabilité identiques à celles de globules rouges initiaux. Après le rescellement, les globules rouges subissent une deuxième série de lavages. Les deux premiers lavages utilisent une solution de sérum physiologique : ils permettent d'éliminer les globules rouges non rescellés.

Les globules rouges sont ensuite remis en plasma autologue à un hématocrite physiologique permettant la transfusion. L'unité de sang transformée est constituée de globules rouges à propriétés oxyphoriques améliorées dont les caractéristiques morphologiques et physiologiques sont proches de globules rouges non transformés.

La mise en oeuvre du procédé de lyse-rescellement permet l'incorporation d'un grand nombre de principes actifs dans des globules rouges, lesquels peuvent être réinjectés au même patient ou à des patients différents. Par exemple l'incorporation d'IHP dans les érythrocytes, permet de modifier la constante d'affinité de l'hémoglobine pour l'oxygène. D'autres applications de ce procédé sont décrites dans les brevets cités précédemment.

Plus particulièrement, la présente invention concerne un dispositif destiné à permettre l'incorporation d'une ou plusieurs substances à activité biologique dans les globules rouges par la technique de lyse-rescellement, tel que décrit dans la revendication 1.

Il faut entendre par "conçu pour un usage unique", que même si les éléments sont destinés à être réutilisés, ils ne le seront qu'après stérilisation et/ou inactivation virale mais seront conçus comme des éléments à usage unique, c'est-à-dire pouvant aisément être mis en place et démonté et de faible coût.

Dans le dispositif de la présente invention, le module de lyse A est constitué
- d'un ensemble assurant l'acheminement d'un tampon de lyse (2) jusqu'à l'entrée de la cartouche de dialyse et l'éventuelle évacuation (3) dudit tampon de lyse, ainsi que des éléments permettant le maintien du module à une température déterminée, notamment 4°C,
- d'un ensemble amovible de préférence à usage unique, comportant une cartouche de dialyse (9) susceptible d'être fixée sur l'arrivée du tampon de lyse pour alimenter l'un des compartiments, l'autre compartiment de la cartouche étant relié à l'extrémité veineuse à un récipient (6) destiné à la mise en température de la suspension d'érythrocytes, et à l'extrémité artérielle à un récipient de stockage (11).

Le module de rescellement est constitué d'un ensemble d'éléments permettant le maintien du module à une température déterminée, de préférence 37°C, et d'un ensemble amovible, de préférence à usage unique et comportant de l'amont vers l'aval, un récipient (22) destiné à assurer la mise en température de la suspension relie a un récipient (26) comportant une amenée de solution de rescellement (28).

L'ensemble de ces modules comporte une pompe permettant l'entrée de la suspension d'érythrocytes dans le module de lyse, une pompe permettant la sortie de la suspension d'érythrocytes du module de rescellement, et une pompe intermédiaire assurant la circulation entre le module de lyse et le module de rescellement. Ces pompes sont de préférence des pompes péristaltiques, qui évitent tout contact avec la suspension.

Le dispositif comporte en outre de préférence en avant de l'unité de rescellement, une tubulure d'amenée d'une solution d'un principe actif à internaliser par exemple de l'ATP (adénosine triphosphate). Pour l'internalisation, suivant la substance à internaliser, celle-ci peut être introduite avec la suspension d'erythrocytes avant la lyse (cas de l'IHP), ou bien ajoutée après la lyse à 4°C (cas de l'ATP) . Les récipients destines a assurer la mise en température des suspensions, sont de préférence des poches plastiques comportant des chicanes à l'intérieur. Ces poches, de préférence allongées, sont placées verticalement et alimentées par le haut et prélevées par le bas.

Le chauffage et la réfrigération des modules peuvent être assurés par une plaque support, qui sur une face recevra les éléments à usage unique et l'autre face sera en contact avec un élément de réfrigération ou de chauffage. De même, dans le cas du module de lyse, le tampon de lyse sera refroidi par passage dans un système de refroidissement approprié.

Le dialyseur lui-même peut être utilisé comme un échangeur de chaleur entre le sang et le tampon de lyse qui y transitent, sans avoir recours à d'autres artifices. Ces échanges de chaleur permettent un traitement particulier des hématies.

Les divers modules placés sur ces plaques, dont la forme sera de préférence adaptée pour fixer les éléments amovibles, pourront recevoir un couvercle et un revêtement qui assurera le maintien des températures de préférence à 4°C et 37°C.

L'élément de lavage peut revêtir différentes formes ; il sera de préférence du type bol séparateur, il pourra également être réutilisé pour effectuer un lavage après traitement de la suspension.

L'élément de lavage est, dans son principe, un réservoir ayant une symétrie de révolution et constitué de deux enveloppes en tronc de cônes concentriques, la chambre de séparation proprement dite étant l'espace compris entre les deux surfaces coniques.

Une des particularités du bol séparateur est que son remplissage se fait à sa base et à sa périphérie ; tout produit introduit, si il est moins dense que ceux qui se trouvent déjà dans la chambre de séparation, devra donc circuler à contre-sens de la sédimentation.

Les composants séparés du produit introduit, s'organisent en anneaux concentriques qui se concentrent par la diminution progressive de leur diamètre, et diminution de leur hauteur par la conicité de la chambre de séparation.

Les différents fluides circulent et se dirigent vers ce bol grâce à une pompe péristallique et huit clamps pneumatiques. Un détecteur d'air, deux capteurs optiques et deux roues codeuses placées, l'une sur l'axe de la pompe, et l'autre sur l'axe de la centrifugeuse, permettent la prise d'information nécessaire à la gestion de l'ensemble des périphériques que sont la pompe, la centrifugeuse et les clamps.

Des éléments aux fonctions du même type sont déjà commercialisés notamment par la société Haemonetics sur des machines V5O/PCS/CELL SAVER®, par exemple le laveur COBE 2997, les séparateurs COBE Spectra, DIDECO Vivacell, BAXTER CS 3000 et Autopheresis PC.

La description ci-après représente un mode de réalisation du dispositif selon l'invention, en utilisant de préférence un séparateur de la société Haemonetics V5O®.

La figure 1 est un schéma général du dispositif à usage unique;

La figure 2 est un schéma du bol séparateur.

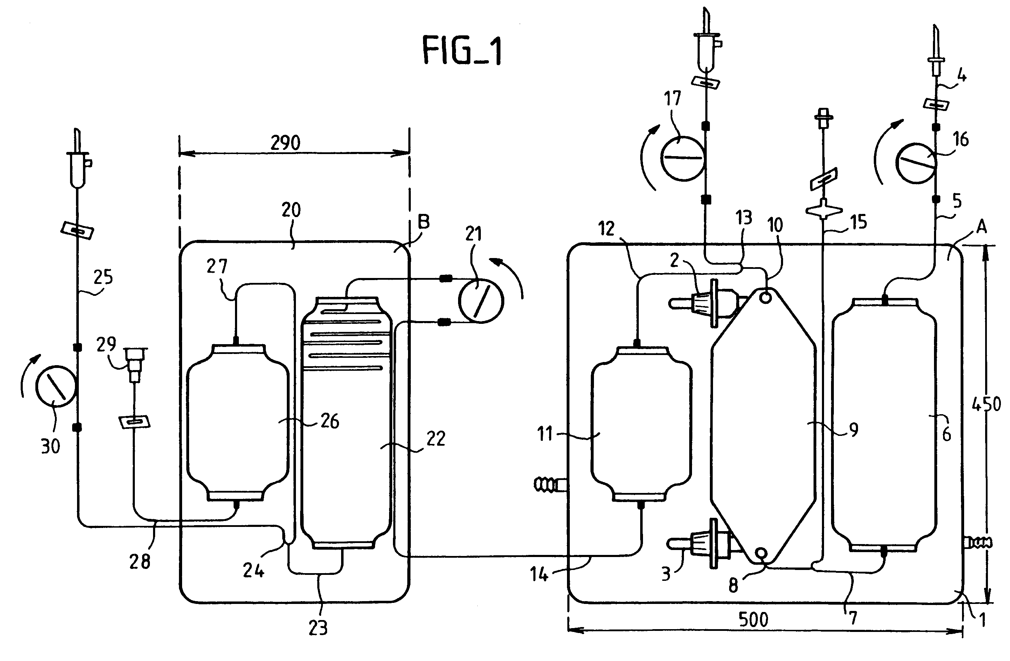

Le dispositif de lyse-rescellement représenté à la figure 1 comporte une unité de lyse A et une unité de rescellement B.

L'unité de lyse A est constituée d'un évaporateur se trouvant au contact d'une plaque métallique (1) en acier inox ayant la forme des éléments du kit à usage unique. L'autre côté de l'évaporateur non présenté et au contact de l'échangeur de chaleur non jetable, contient un serpentin métallique. En fonctionnement, une seconde plaque métallique réfrigérée ou bien simplement en matière isolante articulée sur la première enferme le kit à usage unique dans un volume minimal réfrigéré. Ce boîtier ainsi constitué, impose par ailleurs aux différentes poches et tubulures, leur volume maximum.

Cette plaque métallique (1) comporte deux éléments permettant l'arrivée (2) et le départ (3) du tampon de lyse, dont le système d'alimentation n'est pas représenté, mais est constitué essentiellement par une pompe, un circuit de réfrigération et un bac d'alimentation. Sur la plaque (1) se trouve fixée de manière amovible, la partie dénommée kit à usage unique, de ce module de lyse. Cette partie est composée en se déplaçant de l'entrée du sang vers la sortie du sang traité : d'un perforateur (4) suivi d'une tubulure en PVC (5) qui débouche dans une poche spéciale soudée en forme de serpentin (6), et jouant le rôle d'échangeur thermique ; un serpentin fait d'un tube INOX 1/8 OD de 1 à 1,5 m pourrait être utilisé en lieu et place de la poche PVC en forme de serpentin, pour les échanges de chaleur avec le sang. L'autre extrémité de cet échangeur est lui-même raccordé à une tubulure (7) qui se connecte par un luer mâle spécial à l'entrée artérielle (8) de la cartouche de dialyse (9). A la sortie veineuse (10) du dialyseur, une tubulure (12) mène au haut d'une poche en PVC de 600 ml (11) qui sert de volume tampon, cette tubulure est rejointe par une autre au niveau d'un "Y" (13), entre la sortie du dialyseur (10) et la poche "retard" (11). Finalement une dernière tubulure (14) conduit du bas de cette poche à la sortie du module. L'entrée tampon de lyse du dialyseur est constituée d'une tubulure passant au travers d'une pompe péristaltique et conduisant a un échangeur réalisé par un serpentin métallique non jetable accolé à l'évaporateur. La sortie du dialyseur, en ce qui concerne le liquide de dialyse est quant à elle une simple tubulure débouchant sur un réservoir. Les éléments à refroidir sont donc globalement de deux natures, d'une part le kit à usage unique en matière plastique (PVC, polycarbonate) et le kit réutilisable métallique tout deux au contact intime de la plaque métallique régulée en température, et d'autre part les fluides (sang, tampon de lyse, solution de principe actif) qui circulent et séjournent dans le kit au contact duquel ils acquièrent la température désirée.

Le module de lyse comporte également une tubulure de pompe (15) pour solution additive pouvant être mélangé au sang lysé à 4°C, par exemple l'ATP ou une petite molécule, cette tubulure est raccordée à la tubulure (7). Deux niveaux sont donc possibles pour la mise en présence de la substance active et des globules rouges, avant et immédiatement après la lyse.

Enfin, la circulation du sang est assurée par une pompe péristaltique (16) qui est gérée par un ensemble de commandes comme les autres pompes péristaltiques du dispositif, la pompe (17) placée sur la tubulure (18) qui se raccorde au "Y" (13) est essentiellement destinée à assurer la purge de la bobine de dialyse (9), cette pompe est comme la précédente, gérée par l'unité de commande centrale.

L'apport de principe actif par la tubulure (15) peut être réalisé soit à la main, soit par l'intermédiaire d'une pompe qui n'est pas représentée.

Le module de rescellement B est lui aussi constitué d'un boîtier métallique (20), muni d'une porte non représentée se refermant sur un kit à usage unique, ce boîtier métallique qui au contact d'une source de chaleur assurera le maintien à 37° du module en cause. Le kit à usage unique est composé en se déplaçant de l'entrée du sang à 4 degrés vers la sortie du sang réchauffé, d'une tubulure (14) provenant directement du module "4 degrés", et qui permet le pré-réchauffage du sang. Cette tubulure quitte momentanément le module pour passer dans le corps d'une pompe à sang (21), avant de se raccorder à un échangeur de chaleur identique (22) à celui utilisé dans le module à 4 degrés. La tubulure (23) qui sort de l'échangeur, lui placé dans le module à 37 degrés, rejoint un "Y" (24) dont une des dérivations se termine par un perforateur mâle (25) (à connecter au flacon de rescellement). L'autre dérivation du "Y" conduit à une poche PVC de 600 ml (26). Cette poche possède deux tubulures d'accès opposées (27 et 28), cela permettant au premier sang entré, d'être aussi le premier sorti, elle est identique à la poche "retard" du module "4 degrés". Une tubulure terminée par un embout femelle (29) pour perforateur constitue la sortie de cette poche "retard.

Enfin, l'amenée de la solution de rescellement effectuée par l'intermédiaire de la tubulure (25) et de la pompe (30) est également asservie comme les pompes péristaltiques précédentes à l'unité de commande centrale. Comme on peut le constater à la lecture de cette figure, l'ensemble des tubulures des quatres poches (6) (11) (22) et (26) et la bobine de dialyse (9) qui sont les éléments en contact avec la suspension de globules rouges, sont placés de façon amovible dans les deux unités de rescellement, et peuvent être en conséquent, soit re-stérilisés, ou bien jetés après usage.

Les poches de mise en température sont en PVC, et constituées de deux feuilles quasiment rectangulaires et identiques en PVC soudées entre elles en forme de serpentin. Ce serpentin conduit d'une tubulure (5) en PVC située sur le haut de la poche, à une seconde (7), qui elle est soudée au bas de la poche.
Au sein du boîtier fermé dans lequel la poche est enfermée, la distance maximale entre les faces internes de la poche sera imposée et de l'ordre de de quelques dixièmes de mm. De cette manière, le volume de l'échangeur sera faible (de 10 à 20 ml) et la surface au contact avec la plaque (1) à 4 degrés sera maximum.

La cartouche de dialyse (9) comporte un boîtier parallélipipédique en matière plastique rigide. Ce boîtier est séparé en deux compartiments par des membranes semi-perméables, chacun de ces compartiments possède deux tubes d'entrée-sortie. Un des compartiments est traversé par du sang, l'autre par le tampon de lyse. Le montage de cet élément dans le module est fait de telle façon que le sang circule du bas vers le haut afin d'assurer une purge naturelle du compartiment sang.

La cartouche de dialyse peut aussi être de type à "fibres creuses", où les deux compartiments sont constitues pour le compartiment sang, de l'intérieur de fibres creuses (en matériaux semi-perméables) et pour le compartiment tampon de lyse, du volume extérieur aux fibres et intérieur au boitier rigide renfermant ces fibres.

La poche tampon de sortie de dialyse (11) est une poche de transfert de 600 ml de volume maximal, enfermée dans un volume de 500 ml environ dans lequel elle augmente de volume au cours de son remplissage. Elle a ceci de spécial que la turbulure d'alimentation et celle de vidange sont montées toutes les deux sur un petit côté de la poche, mais de façon opposée. Pour une position verticale de la poche, le premier sang entré (par le haut) sera aussi le premier sorti (par le bas).

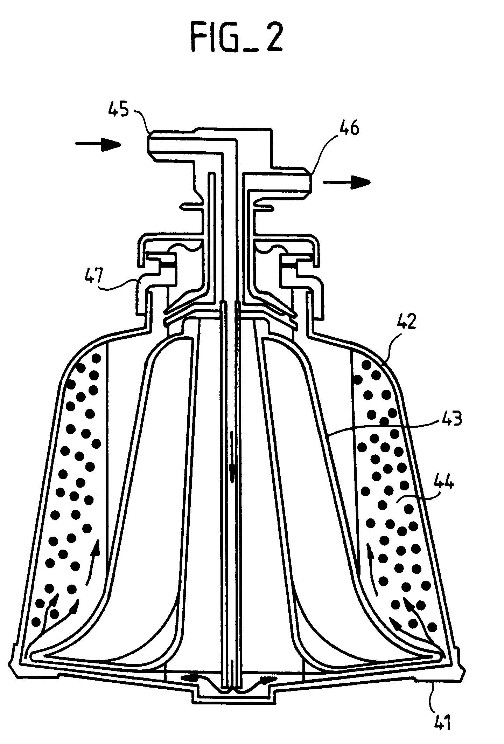

La figure 2 représente la coupe de l'unité de lavage, la description de ce bol réparateur ne sera pas faite en détail puisqu'il s'agit d'un produit qui peut se trouver dans le commerce, et qui a été adapté pour la présente utilisation.

Sur la vue en coupe de la figure 2, on voit que le bol séparateur (41) est dans son principe constitué de deux enveloppes rigides en troncs de cône concentriques (42) et (43). La première est extérieure (42), la seconde (43) placée sur le même axe est incluse dans la précédente. La chambre de séparation (44) est l'espace compris entre les deux surfaces coniques. Ce bol est muni d'un système injecteur (45) et d'un système collecteur (46), séparés de la chambre de séparation par un joint tournant (47) au sommet des deux troncs de cône.

Le réservoir durant la séparation des cellules de la suspension introduite est mis en rotation par une centrifugeuse, le système injecteur et collecteur est quant à lui maintenu par deux bras articulés en position fermée.

L'injecteur (45) par sa connexion à un réseau de tubulures, permet l'introduction dans le séparateur (44) de la suspension de cellules à séparer, ou lors de l'arrêt du bol la vidange de ce dernier.

Le collecteur (46), quant à lui, permet le recueil des constituants séparés, qui se présentent à lui séquentiellement, poussés au dehors par la suspension introduite. Le collecteur est lui aussi connecté à un réseau, qui se réduit à une tubulure qui rejoint une poche à déchet.

Les éléments solides de la suspension sont progressivement concentrés par centrifugation vers l'extérieur de la chambre (44). Un système de détection préalablement calibré avant la centrifugation permet lorsque la concentration en globules rouges a atteint un volume déterminé, et/ou lorsque les globules rouges ont été lavés convenablement, d'obtenir une suspension de globules rouges présentant les caractéristiques souhaitées.

Les exemples ci-après sont destinés à mettre en évidence d'autres avantages et caractéristiques de la présente invention et ne la limitent en aucune manière.

### Exemple 1 : Séparation des hématies du plasma

La séparation des hématies est effectuée au cours du premier lavage, appelé lavage I, et permet le traitement du sang, avant l'étape de la lyse réversible des hématies et leur rescellement.

Le lavage I réalisé sur une unité de sang complet comprend les étapes suivantes :
- la déplasmatisation
- la déleucocytation
- l'élimination des plaquettes
- l'ajustement de l'hématocrite
- la mise en suspension des hématies dans la solution de produit à internaliser (IHP).
- le contrôle de l'osmolarité.

Ces opérations aujourd'hui manuelles, sont réalisées grâce à la présente invention de façon automatique. Lorsqu'une quantité importante de sang doit être traitée, ce lavage peut comporter plusieurs cycles.

### Séquence des opérations

Une suspension cellulaire de 40 % d'hématocrite environ, est préparée par ajout de sérum physiologique à partir d'un concentré érythrocytaire, cela de façon automatique et simultanée à l'introduction du concentré érythrocytaire dans le bol, par alternance d'une quantité d'eau et de sang dans une proportion calculée par la machine de façon à obtenir 40% d'hématocrite.

Le culot dilué est introduit dans le bol décrit à la figure 2 jusqu'à détection optique ou vacuité de la poche, soit un volume d'environ 480 cc, cela à un débit de la pompe à sang de 100 cc/min (réglable de 0 à 250 ml/min) et une vitesse rotation de la centrifugeuse de 6000 t/min. La solution surnageante est recueillie dans une poche à déchets de 5 litres. C'est au cours de cette étape que l'hématocrite est empiriquement déterminé par arrêt du remplissage du séparateur à un niveau de l'interface surnageant/cellules déterminé (42 ml), cela dans des conditions de débit et de centrifugation précises.

Le lavage des hématies est effectué par l'introduction dans le bol d'un volume programmé de solution de NaCl à 9 %, cela à un débit variable, sous le contrôle de l'optique de la tubulure. Le débit de la pompe est asservi de telle manière que le liquide quittant le bol soit à un hématocrite de l'ordre de 1%, permettant l'élimination du "buffy coat" tout en minimisant la perte de globules rouges. Cette étape concerne l'achèvement de la déplasmatisation, ainsi que l'élimination des plaquettes et des globules blancs.

Le sang étant maintenant sous forme d'une suspension de globules rouges "purs" dans la solution de lavage, la mise en suspension en solution d'IHP est réalisée, cela par la répétition (quatre fois) de la manipulation suivante :
- un petit volume d'IHP est introduit dans le bol (de l'ordre de 50 ml à 38 mM/l d'IHP), ce qui déplace la solution de lavage vers la poche à déchets, puis la centrifugeuse est arrêtée de façon à homogénéiser l'IHP dans la solution de suspension des globules rouges, immédiatement suivi d'une remise en marche de la centrifugeuse et d'une attente d'une minute environ pour obtenir une sédimentation correcte.

Lorsque le nombre de lavages souhaités est atteint, la vidange du bol dans une poche de transfert (150 ml/min) peut alors intervenir.

Dans le cas ou le volume de sang à traiter ne suffit pas à l'obtention d'un hématocrite de 72 % avant déleucocytation dans le séparateur (bol de 265 cc), c'est-à-dire si le volume d'hématies est inférieur à 191 ml, la machine se mettra en alarme (détection d'air). L'utilisateur aura dans cette situation le choix entre trois alternatives :
- continuer le lavage avec un hématocrite anormal
- connecter une autre unité et continuer normalement le remplissage du bol
- si aucun autre sang que celui déjà en IHP n'est disponible on utilise celui-ci pour achever le remplissage du bol à 72 % d'hématocrite. Le lavage peut alors s'achever comme précédemment décrit.

Le lavage I est alors terminé et la phase de dialyse peut commencer.

### Exemple II : Etape de lyse et rescellement

Cette phase de lyse et rescellement intervient après le lavage I et s'adresse donc à une suspension d'hématies (70 %) dans une solution d'IHP de volume variable, de 265 cc minimum à 1,3 litre environ. Cette suspension subira les opérations suivantes :
- abaissement de la température à 4°C
- passage dans le dialyseur
- mélange avec une solution d'ATP
- attente à 4 degrés durant 10 minutes environ
- réchauffement à 37°C
- mélange à la solution de rescellement
- attente du lavage II à 37°C de 30 minutes environ.

### Séquence des opérations

La suspension d'hématies provenant du lavage I est entraînée par une pompe péristaltique (16) au travers d'un échangeur de chaleur (6) où sa température s'abaisse à 4°C. Ce débit est réglable de 0 à 32 cc/min au pas de 1ml/min, et détermine le temps de transit du sang dans le dialyseur (9) pour un volume mort donné du compartiment sang). La température quant à elle est réglable de 1°C à la température ambiante. Cette opération de pompage est menée de façon ininterrompue jusqu'à ce que le détecteur d'air d'entrée de pompe soit active, indiquant la vacuité de la poche de sang à traiter. A ce moment là, et afin de permettre la vidange, même partielle, de l'échangeur de chaleur, puis de la cartouche de dialyse, de l'air stérile sera pompé pour un volume fixe.

Les globules rouges, une fois refroidis dans l'échangeur de chaleur (en suspension dans la solution d'IHP) traversent le compartiment sang d'un dialyseur au débit imposé par la programmation de l'utilisateur. La température du dialyseur est contrôlée (4°C). La pression trans-membranaire est mesurée. Le débit de la pompe à tampon de lyse (non représentée) est régulé de manière à maintenir la pression trans-membranaire constante et au niveau désiré, de 0 à 300 mmHg. Le tampon de lyse est lui-même maintenu à 4°C par passage dans un échangeur de chaleur non jetable.

En sortie du dialyseur, et toujours à 4°C, la suspension cellulaire obtenue est mélangée grâce à la tubulure (17) à une solution d'ATP de façon proportionnelle réglable de 1/20 ème à 1/5 ème (rapport pré-réglé à 1/10ème), puis laissée en attente pour 15 minutes maximum, (délai réglable de 0 à 20 min) dans la poche (11). La pompe (17) est chargée de l'acheminement de la solution d'ATP, sa gamme de débit s'étend de 0 à 16 ml/min. Après cela, la suspension est pompée à un débit réglable de 0 à 32 ml/min au pas de 1 ml/min, et portée à 37°C par passage dans un échangeur de chaleur. Cela est accompli par la pompe (21) située après l'échangeur, de façon à travailler sur une portion de tubulure qui ne soit pas rigidifiée par une température trop basse. Puis proportionnellement au débit sanguin et dans un rapport de 1/5 ème à 1/20 ème, le produit de rescellement est mélangé dans la poche (26) au sang réchauffé à 37 degrés après passage dans la poche de réchauffage (22). Une pompe péristaltique est utilisée à cet effet, la (21), sa gamme de débit s'étend de 0 à 16 ml/min.

L'ultime étape est une phase d'attente du produit obtenu, afin d'obtenir le rescellement complet des hématies. La suspension d'hématies est maintenue de 15 à 30 mn à la température de 37°C dans la poche de 600 ml (26) environ. Après cela le lavage II peut alors être réalisé.

### Exemple III : Lavage des hématies après rescellement

L'objet du deuxième lavage est de laver et resuspendre dans du plasma la suspension cellulaire obtenue après la phase de lyse et rescellement. Les étapes suivantes le composent :
- remplissage du bol et élimination de la solution resuspendant les hématies, cette solution contenant essentiellement de l'IHP, de l'ATP et de l'hémoglobine libre.
- élimination des stromas de cellules.
- mise en suspension des hématies traitées dans une solution de conservation, du plasma ou une solution de macromolécules de synthèse.

### Séquence des opérations.

La suspension de cellules traitées par dialyse est introduite dans le bol déjà utilisé pour le lavage I à un débit de 100 cc/min, diluée par de l'eau physiologique dans un rapport de 1:2 et une vitesse de rotation de la centrifugeuse de 5000 trs/min. Cette phase est arrêtée après qu'un volume de 1 l environ ait été introduit, ou qu'une détection d'air dans la tubulure d'alimentation du bol se soit produite, signe de la vacuité de la poche. Dans le cas d'une détection d'air la question est posée, soit de commencer le lavage, soit d'attendre la disponibilité d'autre sang à laver.

Le lavage peut alors commencer par l'introduction dans le bol d'un volume programmé de solution de NaCl à 9%, cela à un débit variable et pour une vitesse de centrifugation de 6000 trs/min, sous le contrôle de l'optique de la tubulure. Le débit de la pompe est asservi de telle manière que le liquide quittant le bol soit à un hématocrite maximum de l'ordre de 1%, permettant l'élimination des stromas de cellules tout en minimisant la perte de globules rouges. Cette phase élimine une partie de l'hémoglobine libre, sans toutefois que celle qui se trouve entre les hématies ne soit réellement affectée.

La centrifugeuse est alors arrêtée, puis remise en marche aussitôt, de façon à homogénéiser la solution de suspension des globules rouges, avant de recommencer pour un volume déterminé l'introduction de sérum physiologique. Trois étapes de lavage avec 500 ml d'eau physiologique sont ainsi réalisées.

Cette phase d'arrêt et redémarrage de la centrifugeuse est répétée une seconde fois, mais seulement pour deux étapes de 100 ml environ, le sérum physiologique étant remplacé par du plasma.

L'arrêt de la centrifugeuse intervient alors et la vidange du bol est réalisée vers une poche de transfert à un débit de 150 ml/min.

Les lavages I et II peuvent correspondre à un enchaînement de cycles (un ou plus), un cycle correspondant à un ensemble de phases et d'étapes.

## Revendications

1. Dispostif destiné à permettre l'incorporation d'une ou de plusieurs substances à activité biologique dans les globules rouges par la technique de lyse-rescellement, le dispositif comportant
(i) un bol de décantation permettant, à partir d'un sang complet ou non, d'obtenir une suspension d'hématies après notamment :
- déplasmatisation
- déleucocytation
- élimination des plaquettes
- ajustement de l'hématocrite
- mise en suspension des hématies dans la solution de produit à internaliser
- contrôle de l'osmolarité
(ii) une unité de lyse (A) et rescellement (B) destinée au traitement de la suspension d'hématies, constituée d'un module de lyse à une température inférieure à 10° C et d'un module de rescellement à une température supérieure à 20° C, l'ensemble des éléments de l'unité de lyse et rescellement entrant en contact avec la suspension d'érythrocytes étant contu pour un usage unique.

2. Dispositif selon la revendication 1, caractérisé en ce que le module de lyse est constitué
- d'un ensemble assurant l'acheminement d'un tampon de lyse jusqu'à l'entrée de la cartouche de dialyse et l'éventuelle évacuation dudit tampon de lyse, ainsi que des éléments permettant le maintien du module à une température déterminée, notamment 4°C,
- d'un ensemble amovible de préférence à usage unique, comportant une cartouche de dialyse susceptible d'être fixée sur l'arrivée du tampon de lyse pour alimenter l'un des compartiments, l'autre compartiment de la cartouche étant relie à l'extrémité veineuse à un récipient destiné à la mise en température de la suspension d'érythrocytes, et à l'extrémité artérielle à un récipient de stockage.

3. Dispositif selon l'une des revendications 1 et 2, caractérisé en ce que le module de rescellement est constitué d'un ensemble d'éléments permettant le maintien du module à une température déterminée, de préférence 37°C, et d'un ensemble amovible, de préférence à usage unique et comportant de l'amont vers l'aval, un récipient destiné à assurer la mise en température de la suspension reliée à un récipient comportant une amenée de solution de rescellement.

4. Dispositif selon l'une des revendications 1 à 3, caractérisé en ce que une pompe péristaltique est disposée afin d'acheminer la suspension traitée de la sortie du module de lyse à l'entrée du module de rescellement.

5. Dispositif selon l'une des revendications 1 à 4, caractérisé en ce que le dispositif comporte en outre deux pompe, péristaltiques qui assurent l'entrée de la suspension d'érythrocytes à traiter dans le module de lyse et la sortie du module de rescellement.

6. Dispositif selon l'une des revendications 1 à 5, caractérisé en ce que les récipients des différents modules sont des poches en matière plastique.

7. Dispositif selon l'une des revendications 1 à 6, caractérisé en ce que le récipient destiné à la mise en température de la suspension d'érythrocytes est une poche à chicane.

8. Dispositif selon l'une des revendications 1 à 7, caractérisé en ce que les poches sont disposées verticalement et sont alimentées par le haut, et prélevées par le bas.

9. Dispositif selon d'une des revendications 1 à 8, caractérisé en ce que la cartouche de dialyse est alimentée par le bas, par la suspension d'érythrocytes.

10. Dispositif selon l'une des revendications 1 à 9, caractérise, en ce que le dispositif comporte des moyens permettant le lavage de la suspension d'érythrocytes après rescellement.

11. Dispositif selon l'une des revendications 1 à 10, caractérisé en ce que le moyen de lavage de la suspension après rescellement est le même que le moyen de lavage avant l'étape de lyse.

12. Dispositif selon l'une des revendications 1 à 11, caractérisé en ce qu'il comporte dans le module de lyse des moyens d'amenée d'une solution dans le circuit.

13. Dispositif selon l'une des revendications 1 à 12, caractérisé en ce que le dispositif comporte des moyens de purge des circuits.

14. Dispositif selon l'une des revendications 1 à 13, caractérisé en ce que chaque module est isolé thermiquement.

15. Dispositif selon l'une des revendications 1 à 14, caractérisé en ce que les éléments permettant le maintien des modules sont constitués par une plaque métallique permettant sur une face, la fixation des éléments amovibles, et en contact sur l'autre face avec un circuit de réfrigération ou de chauffage respectivement.

16. Dispositif selon l'une des revendications 1 à 15, caractérisé en ce qu'il est construit auteur d'un séparateur du type V5O/PCS/CELL SAVER® ou de tout autre type de séparateur.

## Claims

1. Device intended to allow the incorporation of one or more biologically active substances into red blood cells by the lysis-resealing technique, the device comprising
(i) - a settling bowl which permits, starting with a blood, whole or otherwise, a suspension of red blood corpuscles to be obtained after especially:
- removal of plasma
- removal of leucocytes
- removal of platelets
- adjustment of the haematocrit
- suspension of the red blood corpuscles in the solution of product to be internalized
- control of the osmolarity.
(ii) a lysis (A) and resealing (B) unit, intended for the treatment of the suspension of red blood corpuscles, consisting of a lysis module at a temperature less than 10°C and a resealing module at a temperature greater than 20°C, all the elements of the lysis and resealing unit entering into contact with the suspension of erythrocytes being designed for a single use.

2. Device according to Claim 1, characterized in that the lysis module consists
- of a set ensuring the transport of a lysis buffer up to the inlet of the dialysis cartridge and the optional discharge of the said lysis buffer, as well as the elements which make it possible to maintain the module at a determined temperature, especially 4°C,
- of a removable set preferably for single use, incorporating a dialysis cartridge capable of being attached to the lysis buffer feed in order to supply one of the compartments, the other compartment of the cartridge being connected at the venous end to a vessel intended for adjusting the temperature of the erythrocyte suspension, and at the arterial end to a storage vessel.

3. Device according to one of Claims 1 and 2, characterized in that the resealing module consists of a set of elements which allow the module to be maintained at a determined temperature, preferably 37°C, and a removable set, preferably for single use and incorporating from upstream to downstream, a vessel intended to ensure adjustment of the temperature of the suspension, connected to a vessel incorporating an access for resealing solution.

4. Device according to one of Claims 1 to 3, characterized in that a peristaltic pump is placed in order to transport the treated suspension from the outlet of the lysis module to the inlet of the resealing module.

5. Device according to one of Claims 1 to 4, characterized in that the device incorporates, in addition, two peristaltic pumps which ensure the entry of the erythrocyte suspension to be treated into the lysis module and the exit from the resealing module.

6. Device according to one of Claims 1 to 5, characterized in that the vessels of the different modules are pouches made from plastic material.

7. Device according to one of Claims 1 to 6, characterized in that the vessel intended for adjusting the temperature of the erythrocyte suspension is a pouch with baffles.

8. Device according to one of Claims 1 to 7, characterized in that the pouches are placed vertically and supplied via the top and discharged via the bottom.

9. Device according to one of Claims 1 to 8, characterized in that the dialysis cartridge is supplied via the bottom, with the erythrocyte suspension.

10. Device according to one of Claims 1 to 9, characterized in that the device incorporates means which permit the washing of the erythrocyte suspension after resealing.

11. Device according to one of Claims 1 to 10, characterized in that the means for washing the suspension after resealing is the same as the means for washing before the lysis step.

12. Device according to one of Claims 1 to 11, characterized in that it incorporates in the lysis module means for admitting a solution into the circuit.

13. Device according to one of Claims 1 to 12, characterized in that the device incorporates means for purging the circuits.

14. Device according to one of Claims 1 to 13, characterized in that each module is thermally insulated.

15. Device according to one of Claims 1 to 14, characterized in that the elements which permit the modules to be maintained consist of a metal plate permitting, on one face, the attachment of the removable elements, and in contact, on the other face with a cooling or heating circuit respectively.

16. Device according to one of Claims 1 to 15, characterized in that it is constructed around a separator of the V50/PCS/CELL SAVER® type or any other type of separator.

## Patentansprüche

1. Apparat zur Einbringung von einer oder mehreren bioligisch aktiven Substanzen in rote Blutkörperchen mit Hilfe der Technik der Lyse und Wiederversiegelung, wobei dieser Apparat folgendes aufweist:
(i) ein Dekantiergefäß, welches es erlaubt, auf der Basis eines vollständigen oder nicht vollständigen Blutes eine Erythrozytsuspension herzustellen, und zwar insbesondere nach:
- Entfernung des Plasmas
- Entfernung der Leukozyten
- Entfernung der Blutplättchen
- Justierung der Hämatokritis
- Suspendierung des Erythrozyts in der zu verabreichenden Produktlösung
- Kontrolle der Osmolarität
(ii) eine Einheit für die Lyse (A) und die Wiederversiegelung (B) für die Behandlung der Hämatinsuspension, welche aus einem Lysemodul mit einer Temperatur von weniger als 10° C und einem Modul für die Wiederversiegelung mit einer Temperatur von mehr als 20° C besteht, wobei alle Bauteile der Einheit für die Lyse und die Wiederversiegelung, welche mit der Erythrozytensuspension in Berührung treten, für einen einmaligen Gebrauch bestimmt sind.

2. Apparat nach Anspruch 1,
**dadurch gekennzeichnet**, **daß**
das Lysemodul aus folgendem besteht:
- einer Baugruppe für die Weiterleitung eines Lysetampons bis zum Eingang der Dialysekartusche und die eventuelle Entfernung des Dialysetampons, sowie aus den Elementen, welche es erlauben, das Modul auf einer bestimmten Temperatur, insbesondere auf 4° C, zu halten,
- eine lösliche Baugruppe, welche vorzugsweise für den einmaligen Gebrauch bestimmt ist und die eine Dialysekartusche aufweist, welche am Einlaß des Lysetampons befestigt werden kann, um eine der Kammern zu speisen, während die andere Kammer der Kartusche an ihrem Venenende an einen Behälter angeschlossen ist, welcher für die Temperierung der Erythrozytensuspension bestimmt ist, während sie an ihrem Arterienende an einen Vorratsbehälter angeschlossen ist.

3. Apparat nach einem der Ansprüche 1 und 2,
**dadurch gekennzeichnet**, **daß**
das Modul für die Wiederversiegelung aus einer Gruppe von Elementen besteht, welche es erlauben, das Modul auf einer bestimmten Temperatur, vorzugsweise auf 37° C, zu halten, und aus einer löslichen Gruppe, die vorzugsweise für den einmaligen Gebrauch bestimmt ist und die, von oben nach unten gesehen, einen Behälter für die Temperierung der Suspension aufweist, welcher an einen Behälter angeschlossen ist, der eine Zuleitung für die Wiederversiegelungslösung aufweist.

4. Apparat nach einem der Ansprüche 1 bis 3,
**dadurch gekennzeichnet**, **daß**
eine peristaltische Pumpe vorgesehen ist, um die behandelte Lösung von dem Ausgang des Lysemoduls an den Eingang des Wiederversiegelungsmoduls zu leiten.

5. Apparat nach einem der Ansprüche 1 bis 4,
**dadurch gekennzeichnet**, **daß**
dieser Apparat außerdem zwei peristaltische Pumpen aufweist, welche für die Einleitung der in dem Lysemodul aufzubereitenden Erythrozytensuspension und deren Auslaß aus dem Wiederversiegelungsmodul sorgen.

6. Apparat nach einem der Ansprüche 1 bis 5,
**dadurch gekennzeichnet**, **daß**
die Behälter der verschiedenen Module Beutel aus Kunststoff sind.

7. Apparat nach einem der Ansprüche 1 bis 6,
**dadurch gekennzeichnet**, **daß**
der Behälter für die Temperierung der Erythrozytensuspension ein Beutel mit einer Drosselsperre ist.

8. Apparat nach einem der Ansprüche 1 bis 7,
**dadurch gekennzeichnet**, **daß**
diese Beutel vertikal angeordnet sind und von oben befüllt und von unten entnommen werden.

9. Apparat nach einem der Ansprüche 1 bis 8,
**dadurch gekennzeichnet**, **daß**
die Dialysekartusche von der Unterseite mit der Erythrozytensuspension befüllt wird.

10. Apparat nach einem der Ansprüche 1 bis 9,
**dadurch gekennzeichnet**, **daß**
dieser Apparat Mittel aufweist, welche ein Waschung der Erytrhozytensuspension nach der Wiederversiegelung ermöglichen.

11. Apparat nach einem der Ansprüche 1 bis 10,
**dadurch gekennzeichnet**, **daß**
das Mittel für die Waschung der Suspension nach der Wiederversiegelung das gleiche ist, wie das Mittel für die Waschung vor dem Lyseschritt.

12. Apparat nach einem der Ansprüche 1 bis 11,
**dadurch gekennzeichnet**, **daß**
sie in dem Lysemodul Mittel für die Einspeisung einer Lösung in den Kreislauf aufweist.

13. Apparat nach einem der Ansprüche 1 bis 12,
**dadurch gekennzeichnet**, **daß**
die Apparat Mittel für die Spülung der Kreisläufe aufweist.

14. Apparat nach einem der Ansprüche 1 bis 13,
**dadurch gekennzeichnet**, **daß**
jedes der Module thermisch isoliert ist.

15. Apparat nach einem der Ansprüche 1 bis 14,
**dadurch gekennzeichnet**, **daß**
die Vorrichtung für die Halterung der Module aus einer Metallplatte besteht, auf deren einer Seite die löslichen Elemente befestigt sind, während sie mit ihrer anderen Seite mit einem Kühlkreis, bzw. mit einem Heizkreis in Berührung steht.

16. Apparat nach einem der Ansprüche 1 bis 15,
**dadurch gekennzeichnet, daß**
sie um einen Separator vom Typ V50/PCS/CELL SAVER® oder eine beliebige andere Art eines Separators angeordnet ist.
